# FASCICULE DE BREVET EUROPEEN

(11) **EP 0 906 765 B1**
(45) Date de publication et mention de la délivrance du brevet: **30.07.2003**
(21) Numéro de dépôt: 98402313.5
(22) Date de dépôt: 18.09.1998
(51) Int. Cl.: A61M 15/08

(54) **Dispositif d'inhalation par voie nasale**
Nasale Inhaliervorrichtung
Nasal inhalation device

(30) Priorité: 03.10.1997 FR 9712349
(43) Date de publication de la demande: 07.04.1999
(73) Titulaire: L'OREAL, 75008 Paris (FR)
(72) Inventeur: Gueret, Jean-Louis H., 75018 Paris (FR)
(74) Mandataire: Boulard, Denis

(56) Documents cités:
- WO-A-88/01884
- CH-A- 492 458
- FR-A- 945 897
- FR-A- 2 654 002
- GB-A- 520 491
- US-A- 3 788 296
- US-A- 4 955 945

## Description

La présente invention a trait à un dispositif d'inhalation par voie nasale de substances ayant une activité, notamment anti-stress, anti allergique, ou apte à favoriser la respiration

Un objet de l'invention consiste à fournir un dispositif pouvant être accroché sur une partie du nez, de manière à restituer sous forme d'émanations volatiles localisées au voisinage des narines, un ou plusieurs actifs inhalables par voie nasale.

Des dispositifs d'inhalation ont été décrits dans les brevets FR-A 945 897, GB-A-520 491 ou US-A-3 788 296 CH-A-492 458. Dans tous ces dispositifs, le support contenant les actifs est toujours disposé à l'intérieur des narines, voire en contact avec les muqueuses nasales, soit directement en regard des narines. Certains de ces dispositifs sont destinés à être utilisés sur des animaux. Un des problèmes résultant de l'utilisation de ces dispositifs tient au fait que les émanations, dirigées directement à l'intérieur des narines, ou directement en regard des narines, peuvent agresser les muqueuses nasales, et incommoder la personne qui les porte. Le problème est particulièrement sensible lorsque le dispositif contient des substances aromatiques dont le parfum peut être très puissant. En outre, peuvent se poser des problèmes de réactions allergiques ou inflammatoires, du fait de la présence de ces actifs, trop proche des muqueuses nasales.

C'est en particulier un objet de l'invention que de fournir un dispositif d'encombrement réduit, qui peut être porté en provoquant sensiblement aucune gène pour la personne qui le porte, notamment la nuit, en position allongée.

C'est un autre objet de l'invention que de fournir un dispositif apte à permettre une "aromathérapie" utilisant des substances, en particulier à base d'huiles essentielles, contenant notamment du camphre, de l'eucalyptus, du thym, de la lavande, du muguet, pouvant avoir une action anti-stress, anti allergique, une action sur la respiration, ou toute autre action visant à améliorer le confort de la personne qui le porte.

C'est encore un autre objet de l'invention que de réaliser un dispositif formant une structure rechargeable.

C'est encore un autre objet de l'invention que de fournir un dispositif simple et économique à réaliser, et permettant un débit contrôlé des émanations inhalées.

D'autres objets de l'invention apparaîtront de manière détaillée dans la description qui suit.

Selon l'invention, ces objets sont atteints en réalisant un dispositif d'inhalation formé d'au moins un support contenant un produit comportant au moins un actif, ledit support étant apte à restituer sous forme d'émanations, une phase volatile dudit (ou desdits) actif(s), ledit dispositif comprenant des moyens permettant l'accrochage du dispositif en au moins un point situé au voisinage des narines de manière à permettre une inhalation par voie nasale, desdites émanations, le support et les moyens d'accrochage étant agencés de sorte que lesdites émanations ne soient pas dirigées directement au contact ou en regard des narines, caractérise en ce qu'il comprend des moyens pour faire varier le débit des inhalations restituées par le support.

On réalise ainsi un dispositif qui tient automatiquement en place sur le nez, ce qui permet à l'utilisateur de se livrer simultanément à d'autres activités. Le dispositif est d'un confort remarquable. Aucune partie du support apte à restituer directement des émanations, n'est disposée à l'intérieur des narines de l'utilisateur. Dans la pratique, le support est disposé suffisamment proche des narines de manière à ce que le souffle nasal puisse, par sa chaleur, favoriser le dégagement desdites émanations. Toutefois, les émanations ne sont pas directement en contact des narines ou des muqueuses nasales, ni dirigées directement en regard des narines, de manière à ne pas incommoder l'utilisateur. De bons résultats ont été obtenus avec la partie "émanatrice" du support localisée à environ 1 à 2 cm des orifices nasaux. Ceci est particulièrement intéressant lorsque le support contient des huiles essentielles, dont le parfum est relativement agressif, ou d'autres actifs, dont la mise en contact avec les muqueuses nasales ou narines pourrait provoquer des réactions allergiques ou inflammatoires.

Au sens de la présente invention, le terme "actif" définit un composé ayant soit une activité thérapeutique (stress, allergie, respiration, sommeil) ou une action de confort (notamment par des senteurs ou parfums).

Comme mentionné précédemment, le support est maintenu au voisinage des narines, notamment au dessus ou au dessous, de sorte que le souffle chaud de la respiration, outre la température normale du corps de la personne qui le porte, permette de favoriser les émanations volatiles, lesquelles sont ensuite inhalées par voie nasale. L'inhalation est permanente, et, une fois le dispositif mis en place sur le nez, ne nécessite aucun geste particulier.

Avantageusement, lesdits moyens d'accrochage sont formés par au moins une portion d'un corps apte à recevoir ledit support. Cette version offre de nombreuses possibilités en termes de confort, de rechargeabilité du dispositif, de réutilisation du dispositif, etc.

Selon un mode de réalisation avantageux, le dispositif comporte deux portions de bras formant une pince, dont une première extrémité est solidaire du dispositif, une seconde extrémité étant libre, lesdites seconde extrémités étant situées en regard l'une de l'autre, et délimitant un espace apte à permettre l'accrochage du dispositif sur l'arête nasale, sur du cartilage nasal, ou sur l'une ou l'autre des parois latérales délimitant les narines, lesdites portions de bras étant aptes à s'écarter élastiquement lors de l'accrochage du dispositif. La pince est de préférence formée par une portion du corps à l'intérieur duquel est monté le support. Le profil, et les surfaces des parties de la pince en contact d'accrochage, sont choisis de manière à procurer le maximum de confort à son utilisateur.

Selon un mode de réalisation avantageux, le dispositif présente sensiblement à l'opposé d'un point médian de l'espace délimité par lesdites seconde extrémités, une échancrure apte à conférer plus d'élasticité auxdites portions de bras. On augmente ainsi la tenue du dispositif sur le nez, la facilité de pose et d'enlèvement du dispositif, et le confort qu'il procure.

De préférence, ledit corps forme un évidement apte à recevoir ledit support, ledit corps étant constitué d'un matériau étanche auxdites émanations, et comportant au moins une ouverture apte à laisser passer lesdites émanations. Ainsi, on peut localiser de manière très précise les émanations, de manière à ne pas gêner l'utilisateur. On évite en particulier aux émanations d'être inhalées directement depuis le support, ce qui, notamment avec certaines substances fortement aromatiques, peut être particulièrement inconfortable.

Le support peut être monté de manière amovible dans l'évidement de manière à former une structure rechargeable. Le corps peut ainsi être réutilisable de nombreuses fois, en changeant seulement la cartouche formée par le support. On peut notamment commercialiser le dispositif sous forme d'un kit comportant un corps rechargeable, et une pluralité de recharges contenant par exemple plusieurs senteurs, à actions identiques ou différentes. Avantageusement, dans cette hypothèse, ledit corps est constitué d'un matériau lavable.

A titre d'indication, le corps est réalisé en un matériau choisi parmi les polypropylènes, les polyéthylènes, les chlorures de polyvinyle, les élastomères naturels, synthétiques ou thermoplastiques.

Selon un mode de réalisation particulier, le dispositif forme une structure sensiblement plate comportant deux faces principales et une tranche, ladite ouverture étant formée dans l'une et/ou l'autre desdites faces principales et/ou dans la tranche. Plusieurs ouvertures peuvent être prévues de manière à permettre notamment une diffusion plus importante du produit, ou bien une diffusion satisfaisante, quelle que soit la position d'accrochage du dispositif sur le nez, l'une de ces ouvertures pouvant servir spécifiquement à l'insertion d'une recharge. A titre de variante, le dispositif contient deux supports superposés, l'un des supports diffusant au travers d'une première ouverture située sur une première face du dispositif, l'autre diffusant au travers d'une seconde ouverture disposée sur l'autre face du dispositif. On peut réaliser ainsi des "cocktails" d'émanations à actions ou senteurs combinées.

Selon un mode de réalisation avantageux, la position du support dans l'évidement est ajustable de manière à modifier le débit de sortie desdites émanations. A titre d'exemple, la tranche comporte une ouverture pour permettre une insertion à profondeur variable du support dans l'évidement.

Selon une variante, le support est à position fixe dans l'évidement, la taille d'au moins une ouverture étant variable de manière à modifier le débit de sortie desdites émanations. A titre d'exemple, le support forme une structure de type comportant un fond et un couvercle monté en rotation sur le fond, la position angulaire du fond par rapport au couvercle déterminant la taille d'une ouverture formée soit sur la tranche soit sur l'une au moins des faces principales du dispositif.

Selon une autre variante, le support est constitué d'un élément formant une structure définissant un volume fermé apte à contenir le produit, ledit élément étant obtenu de soufflage d'un matériau perméable auxdites émanations. A titre d'exemple, ledit matériau est choisi parmi les polyéthylènes basse densité, les chlorures de polyvinyle souples, les copolymères d'éthylène et d'acétate de vinyle (EVA), etc. Avantageusement, après soufflage, le volume est fermé au niveau d'une zone localisée servant de moyen de préhension du dispositif.

Avantageusement, le support est constitué d'un matériau poreux apte à absorber le produit en profondeur.

Le matériau peut être saturé en produit, par saturation sous vide, par gonflage, par pression, ou par capillarité, et peut être choisi parmi les mousses de NBR (natural butadiène rubber), de SBR (synthetic butadiène rubber), les mousses d'élastomère, notamment de polyéther bloc amide, de polyuréthane, ou de PEBAX®, le feutre, la laine, la cellulose, le bois, la terre cuite, le plâtre, les céramiques, etc..

Le support peut également être constitué d'une matrice constituée d un matériau choisi parmi les silicones, les latex, les polyuréthanes, les gommes, etc..

Selon certains modes de réalisation, l'une au moins des faces du support est recouverte d'une feuille d'un matériau choisi parmi les polyéthylènes, le feutre, etc.. Dans cette configuration, l'une au moins des feuilles peut être imperméable auxdites émanations, et être pelable, de manière à permettre le passage des émanations au travers de la face correspondante du support.

A titre d'indication, le dispositif forme une structure sensiblement plate et présente une épaisseur comprise entre 0,5 mm et 10 mm, et de préférence entre 1 mm et 8 mm, et de préférence encore, entre 1,5 mm et 5 mm. Le support peut être constitué d'une pièce coupée ou moulée.

A titre d'exemple, ledit produit contient une substance aromatique ayant une activité de confort ou thérapeutique, notamment anti-stress ou anti-allergique, ou favorisant le sommeil ou la respiration.

le produit peut être choisi parmi les huiles essentielles, notamment à base de camphre, d'eucalyptus, de lavande, de thym, ou de muguet.

L'invention consiste, mises à part les dispositions exposées ci-dessus, en un certain nombre d'autres dispositions qui seront explicitées ci-après, à propos d'exemples de réalisation non limitatifs, décrits en référence aux figures annexées, parmi lesquelles :
- la figure 1A illustre un dispositif d'inhalation;
- la figure 1B illustre une variante de la figure 1A;
- la figure 2 illustre un mode d'accrochage du dispositif;
- les figures 3A, 3B, et 6 illustrent trois variantes du mode de réalisation du dispositif selon l'invention;
- la figure 4 illustre une seconde variante de la figure 1A; et
- les figures 5A-5B illustrent une vue en coupe de deux modes de réalisation d'un support utilisé dans le dispositif selon la présente invention.

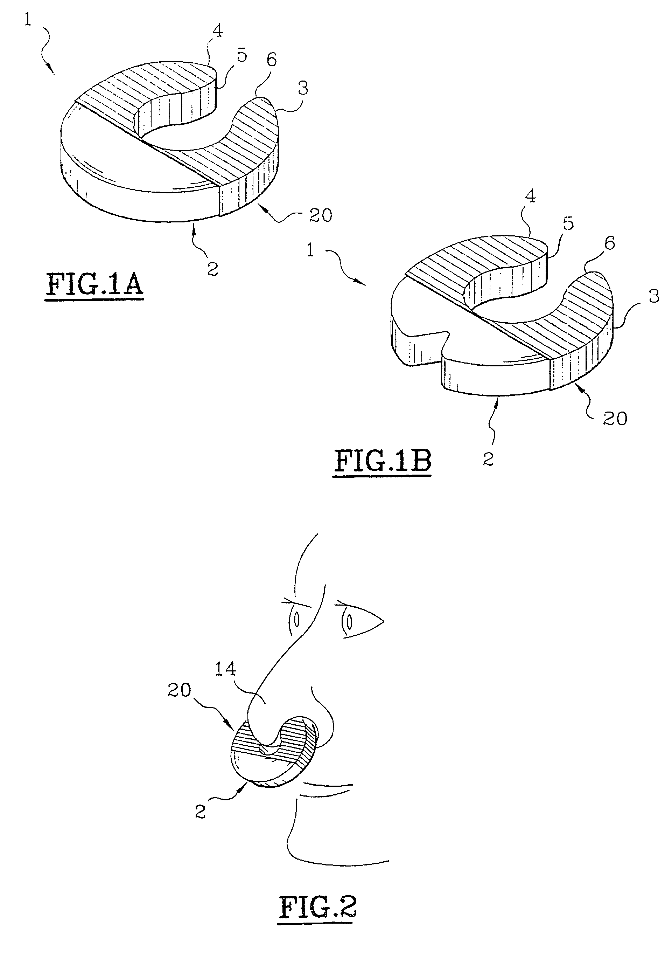

Les exemples correspondant aux figures 1A, B, 2 et 4 ne forment pas part de la présente invention.

Les figures 1A et 1B illustrent un dispositif d'inhalation. Le dispositif 1 forme une structure sensiblement plate, constituée d'un support 2, obtenu de découpe ou de moulage. Le support forme une boucle sensiblement fermée présentant deux bras 3, 4, dont les extrémités libres 5, 6 sont situées en regard l'une de l'autre, et servent à l'accrochage du dispositif sur le nez 14. La partie du support formant les bras d'accrochage 3, 4, est disposée à l'intérieur d'une coque thermoformée 20 réalisée en un matériau étanche aux émanations, de manière à ce que, lorsque le dispositif est monté sur le nez de la manière représentée à la figure 2 les émanations ne soient pas dirigées directement en regard ou au contact des narines. En outre, le support 2 n'est pas disposé directement à l'intérieur des narines, et ne touche pas directement les muqueuses nasales.

Le support 2 est constitué d'un matériau élastiquement déformable, de manière à permettre le montage du dispositif, de la façon illustrée à la figure 2, c'est à dire par accrochage sur le cartilage central séparant les deux narines. Alternativement, le support peut être monté par accrochage sur la paroi latérale de l'une ou l'autre des narines. Lors du montage, les extrémités libres 5, 6, des bras 3, 4, s'écartent élastiquement, et reviennent par rappel élastique en position d'accrochage. Le profil des extrémités libres des bras 3, 4 est choisi de manière à procurer un maximum de confort à l'utilisation. Dans la pratique, une élasticité même très faible, suffit.

Le dispositif est réalisé en un matériau poreux apte à absorber le produit en profondeur. Ledit matériau peut être saturé en produit, par saturation sous vide, par gonflage, par pression, ou par capillarité. A titre d'exemple, le matériau est choisi parmi les mousses de NBR (natural butadiène rubber), de SBR (synthetic butadiène rubber), les mousses d'élastomère, notamment de polyéther bloc amide, de polyuréthane, ou de PEBAX®.

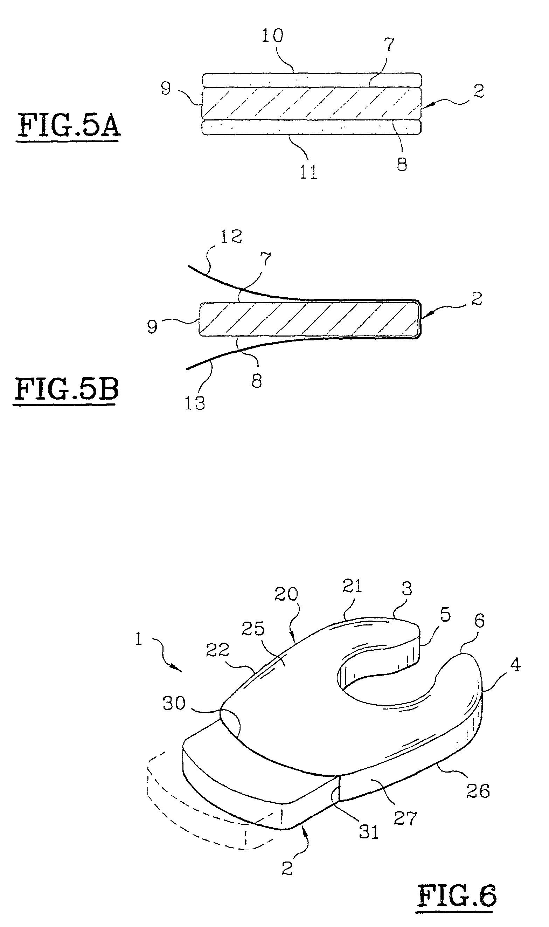

L'élasticité peut résulter de la structure de la coque thermoformée 20, du matériau formant le support 2, ou résulter de l'élasticité de couches ou de feuilles recouvrant les deux faces du support 2. Ainsi par exemple, à la figure 5A, le support 2 est recouvert sur chacune de ses faces 7, 8, d'une feuille 10, 11 d'un matériau tel qu'un polyéthylène ou un caoutchouc, ou du feutre. Outre l'élasticité qu'elles peuvent conférer à la structure du support, de telles feuilles 10, 11, lorsqu'elles sont étanches au produit contenu dans le support 2, permettent de limiter la diffusion du produit seulement par la tranche 9 du support, ce qui peut être souhaitable pour certains produits fortement aromatiques, de manière à ne pas incommoder l'utilisateur. Par ailleurs, ces feuilles facilitent la manipulation du support imbibé de produit liquide. Dans cette configuration, outre les matériaux cités précédemment, d'autres matériaux peuvent être utilisés pour réaliser le support contenant le produit à inhaler. A titre d'exemple, on peut utiliser une matrice en silicone. La matrice peut être rigidifiée en y incluant une trame, notamment en coton.

Dans le mode de réalisation, illustré à la figure 5B, chacune des faces 7, 8, du support 2 est recouverte d'une feuille pelable en polyéthylène 12, 13. Les feuilles 12, 13 sont enlevées avant la première utilisation. Alternativement, les deux feuilles sont enlevées après une certaine période d'utilisation, le produit ne diffusant initialement que par la tranche. Les feuilles sont enlevées avant insertion dans la coque 20, simultanément, ou l'une en premier, puis l'autre après encore un certain temps d'utilisation, lorsque la diffusion par la tranche 9, puis par la première face, devient insuffisante.

Dans la figure 1B, le support 2 présente sensiblement à l'opposé d'un point médian de l'espace délimité par lesdites seconde extrémités libres 5, 6, une échancrure 15 apte à conférer plus d'élasticité auxdites portions de bras 3, 4.

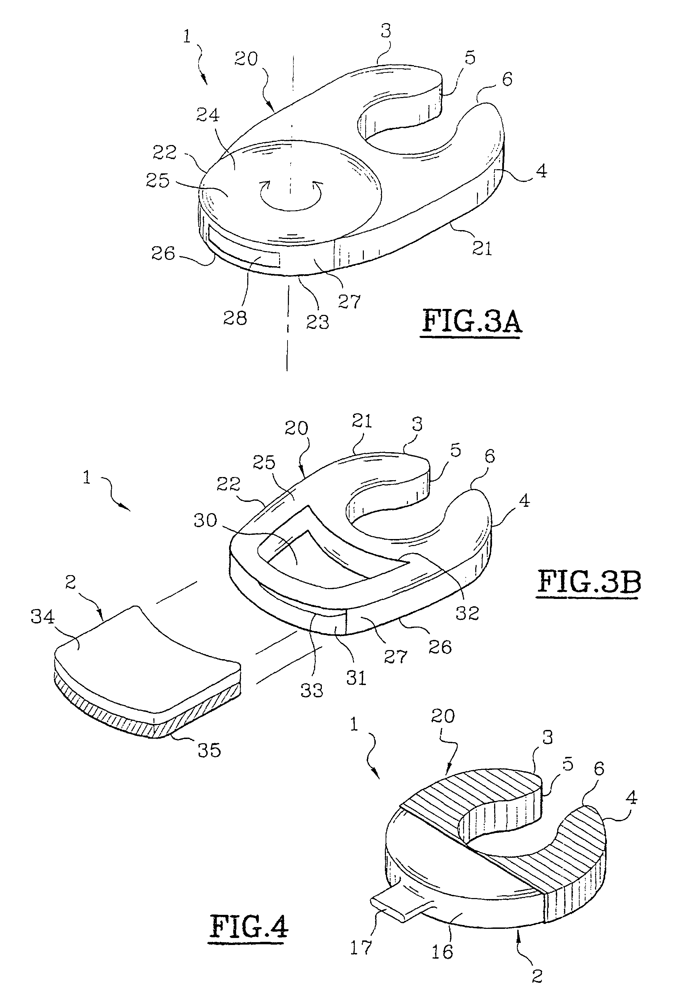

Dans la figure 4, le support 2 est formé d'un élément 16 définissant un volume fermé, obtenu notamment par soufflage, et contenant sous forme liquide, le produit à inhaler. Après remplissage, l'élément 16 est fermé au niveau d'une zone 17 située à l'opposé de l'extrémité libre 5,6, des bras 3, 4, notamment par soudure haute fréquence. Les bras 3, 4 sont disposés chacun à l'intérieur d'une portion thermoformée 20, réalisée en un matériau étanche aux émanations, de manière à éviter toute émanation directement en regard des narines. De la même manière que pour les figures 1A et 1B, l'accrochage se fait en provoquant, lors du montage du dispositif, un léger écartement des bras 3, 4, lesquels bras reviennent par rappel élastique en position initiale, de manière à réaliser l'accrochage du dispositif soit sur l'arête nasale, soit sur le cartilage central délimitant les deux narines, soit sur un des bords latéraux de l'une et/ou l'autre des narines. Avantageusement, la zone 17 fait office de moyen de préhension pour le dispositif 1. Le matériau utilisé pour réaliser l'élément 16 est choisi parmi les matériaux perméables à la phase volatile du produit contenu dans l'élément 16. A titre d'exemple, on utilise un polyéthylène, un chlorure de polyvinyle souple, ou un copolymère d'éthylène et d'acétate de vinyle (EVA). Avant utilisation, la surface de l'élément 16 peut être recouverte d'un élément adhésif étanche à ladite phase volatile.

Dans le mode de réalisation de la figure 3A, le support est contenu à l'intérieur d'un corps 20 réalisé en un matériau étanche à la phase volatile du produit à inhaler. A titre d'exemple, on utilise un matériau lavable, tel qu'un polypropylène. Dans ce mode de réalisation, le corps 20 comporte deux parties 21, 22. La première partie 21 est constituée principalement des bras 3, 4, servant à l'accrochage du dispositif 1 sur le nez, de manière identique aux autres modes de réalisation. La seconde partie 22, montée par exemple par claquage sur la première 21, forme un boîtier constitué d'un fond 23 et d'un couverc e 24, monté libre en rotation sur le fond, et à l'intérieur duquel est disposé un support du type de ceux discutés en référence aux figures qui précèdent. Le boîtier présente deux faces principales 25, 26, et une tranche 27. Le couvercle 24 définit avec le fond 23, une ouverture 28 sur la tranche 27, orientée à l'opposé des narines, et dont la largeur varie en fonction de la position angulaire du fond sur le couvercle, ladite ouverture 28 étant totalement obturée en position de stockage. On joue ainsi sur le débit de sortie des émanations volatiles. Un tel dispositif peut constituer en outre, une structure rechargeable en montant le couvercle 24 de façon amovible par rapport au fond 23. Le support, contenu à l'intérieur du boîtier, peut être réalisé en un matériau qui, outre ceux déjà cités en référence aux figures 1A, 1B, 5A, 5B, peut être choisi parmi le bois, le feutre, la laine, la cellulose, la terre cuite, le plâtre, la céramique, etc..

Dans le mode de réalisation de la figure 3B, le dispositif comprend un corps 20 constitué de deux parties 21, 22, formées d'une seule pièce. La partie 21 comprend essentiellement les bras d'accrochage 3, 4. La seconde partie 22 forme un évidement 30 comportant deux faces principales 25, 26 et une tranche 27. La tranche, présente à l'opposé de l'extrémité libre 5, 6 des bras 3, 4, une ouverture 31 destinée à l'introduction d'un support 2, du type de ceux décrits en référence aux figures précédentes, à la différence près qu'il est formé d'une structure bi-couches 34, 35, contenant chacune un actif ou une senteur différente. Les deux couches peuvent être montées l'une sur l'autre par collage, ou être libres, définissant ainsi deux supports distincts. Chacune des faces 25, 26 présente une ouverture 32, 33, pouvant avant la première utilisation, être obturée par une pellicule occlusive pelable. En cours d'utilisation, après avoir inséré le support 2 dans l'évidement 30, l'utilisateur enlève l'une et/ou l'autre des pellicules occlusives, de manière à permettre une diffusion au travers de l'une et/ou l'autre des ouvertures 32, 33, de manière à inhaler l'un ou l'autre des actifs ou senteurs, ou un cocktail des deux. Ainsi, la diffusion se fait soit au travers de l'ouverture 31 orientée à l'opposé des narines, soit au travers de l'une et/ou l'autre des ouvertures 32, 33 orientées perpendiculairement aux narines lorsque le dispositif est accroché de la façon représentée à la figure 2. A nouveau, aucune émanation n'est dirigée directement en regard des narines.

Dans le mode de réalisation de la figure 6, l'évidement 30 présente une seule ouverture 31 sur la tranche 27, de largeur sensiblement identique à la largeur du support, de manière à permettre une insertion en force du support au travers de ladite ouverture. En cours d'utilisation, l'utilisateur enfonce plus ou moins le support 2 à l'intérieur de l'évidement 30 de manière à jouer sur le débit de sortie des émanations, celui-ci étant d'autant plus faible que le support est enfoncé à l'intérieur de l'évidement 30. Dans la position du support illustrée en traits pleins, la diffusion du produit est plus faible que dans la position illustrée en traits interrompus. A nouveau, les émanations ne sont pas dirigées directement en regard des narines.

Dans la description détaillée qui précède, il a été fait référence à des modes de réalisation préférés de l'invention. Il est évident que des variantes peuvent y être apportées sans s'écarter de l'invention telle que revendiquée ci-après.

## Revendications

1. Dispositif d'inhalation (1) formé d'au moins un support (2) contenant un produit comportant au moins un actif, ledit support étant apte à restituer sous forme d'émanations, une phase volatile dudit (ou desdits) actif(s), des moyens (3-6) permettant l'accrochage du dispositif en au moins un point situé au voisinage des narines de manière à permettre une inhalation par voie nasale, desdites émanations, le support et les moyens d'accrochage étant agencés de sorte que les émanations ne soient pas dirigées directement en regard ou au contact des narines, **caractérisé en ce qu'**il comprend des moyens (31, 28, 23, 24) pour faire varier le débit des inhalations restituées par le support (2).

2. Dispositif selon la revendication 1 **caractérisé en ce que** lesdits moyens d'accrochage sont formés par au moins une portion d'un corps (20) en un matériau étanche aux émanations, et apte à recevoir le (ou les) support(s) (2).

3. Dispositif selon l'une quelconque des revendications 1 et 2 **caractérisé en ce qu'**il comporte deux portions de bras (3, 4) formant une pince, dont une première extrémité est solidaire du dispositif, une seconde extrémité (5, 6) étant libre, lesdites seconde extrémités (5, 6) étant situées en regard l'une de l'autre, et délimitant un espace apte à permettre l'accrochage du dispositif (1) sur l'arête nasale, sur du cartilage nasal, ou sur l'une ou l'autre des parois latérales délimitant les narines, lesdites portions de bras (3, 4) étant aptes à s'écarter élastiquement lors de l'accrochage du dispositif.

4. Dispositif selon la revendication 3, **caractérisé en ce que** le dispositif présente sensiblement à l'opposé d'un point médian de l'espace délimité par lesdites seconde extrémités (5, 6), une échancrure (15) apte à conférer plus d'élasticité auxdites portions de bras (3, 4).

5. Dispositif selon la revendication 2 **caractérisé** en que ledit corps (20) forme un évidement (30) apte à recevoir le (ou les) support(s) (2), ledit corps (20) étant constitué d'un matériau étanche auxdites émanations, et comportant au moins une ouverture (31, 32, 33) apte à laisser passer lesdites émanations.

6. Dispositif selon la revendication 5 **caractérisé en ce que** le support est constitué d'une structure à deux couches superposées (34, 35), ou **en ce que** le dispositif comprend deux supports superposés, le premier support ou la première couche (34) comprenant un premier actif diffusant au travers d'une première ouverture (32) ménagée dans une première face (25) du corps, le second support ou la seconde couche (35) comprenant un second actif diffusant au travers d'une seconde ouverture (33) ménagée dans une seconde face (26) du corps (20), opposée à la première.

7. Dispositif selon la revendication 5 ou 6 **caractérisé en ce que** le (ou les) support(s) (2) est (sont) monté(s) de manière amovible dans l'évidement (30) de manière à former une structure rechargeable.

8. Dispositif selon la revendication 7 **caractérisé en ce que** ledit corps (20) est constitué d'un matériau lavable.

9. Dispositif selon l'une quelconque des revendications 5 à 8 **caractérisé en ce que** le corps (20) est réalisé en un matériau choisi parmi les polypropylènes, les polyéthylènes, les chlorures de polyvinyle, les élastomères naturels, synthétiques ou thermoplastiques.

10. Dispositif selon l'une quelconque des revendications 5 à 9 **caractérisé en ce qu'**il forme une structure sensiblement plate comportant deux faces principales (25, 26) et une tranche (27), ladite ouverture étant formée dans l'une et/ou l'autre desdites faces principales (25, 26) et/ou dans la tranche (27).

11. Dispositif selon l'une quelconque des revendications 5 à 10 **caractérisé en ce que** la position du (ou des) support(s) (2) dans l'évidement est ajustable de manière à modifier le débit de sortie desdites émanations.

12. Dispositif selon les revendications 10 of 11 **caractérisé en ce que** la tranche comporte une ouverture (31) pour permettre une insertion à profondeur variable du (ou des) support(s) (2) dans l'évidement (30).

13. Dispositif selon l'une quelconque des revendications 5 à 10 **caractérisé en ce que** le (ou les) support(s) (2) est (sont) à position fixe dans l'évidement (30), la taille d'au moins une ouverture (28) étant variable de manière à modifier le débit de sortie desdites émanations.

14. Dispositif selon les revendications 10 et 13 **caractérisé en ce que** le corps comporte un fond (23) et un couvercle (24) monté en rotation sur le fond, la position angulaire du fond (23) par rapport au couvercle (24) déterminant la taille d'une ouverture (28) formée soit sur la tranche (27) soit sur l'une au moins des faces principales (25, 26) du dispositif.

15. Dispositif selon l'une quelconque des revendications 1 à 14 **caractérisé en ce que** le support (2) est constitué d'un élément (16) formant une structure définissant un volume fermé apte à contenir le produit, ledit élément étant obtenu de soufflage d'un matériau perméable auxdites émanations.

16. Dispositif selon la revendication 15 **caractérisé en ce que** ledit matériau est choisi parmi les polyéthylènes basse densité, les chlorures de polyvinyle souples, ou les copolymères d'éthylène et d'alcools d'acétate de vinyle (EVA).

17. Dispositif selon l'une quelconque des revendications 1 à 14 **caractérisé en ce que** le (ou les) support(s) (2) est (sont) constitué(s) d'un matériau poreux apte à absorber le produit en profondeur.

18. Dispositif selon la revendication 17 **caractérisé en ce que** ledit matériau est saturé en produit, par saturation sous vide, par gonflage, par pression, ou par capillarité.

19. Dispositif selon la revendication 17 ou 18 **caractérisé en ce que** ledit matériau est choisi parmi les mousses de NBR (natural butadiène rubber), de SBR (synthetic butadiène rubber), les mousses d'élastomère, notamment de polyéther bloc amide, de polyuréthane, ou de PEBAX®, le feutre, la laine, la cellulose, le bois, la terre cuite, le plâtre, ou les céramiques.

20. Dispositif selon l'une quelconque des revendications 1 à 16 **caractérisé en ce que** le (ou les) support(s) est (sont) constitué(s) d'une matrice constituée d'un matériau choisi parmi les silicones, les latex, les polyuréthanes, ou les gommes.

21. Dispositif selon l'une quelconque des revendications précédentes **caractérisé en ce que** l'une au moins des faces (7, 8) du support (2) est recouverte d'une feuille (10-13) d'un matériau en polyéthylène, ou en feutre.

22. Dispositif selon la revendication précédente **caractérisé en ce que** l'une au moins des feuilles (12, 13) est imperméable auxdites émanations, et pelable, de manière à permettre le passage des émanations au travers de la face correspondante (7, 8) du support (2).

23. Dispositif selon l'une quelconque des revendications précédentes **caractérisé en ce qu'**il forme une structure sensiblement plate et présente une épaisseur comprise entre 0,5 mm et 10 mm, et de préférence entre 1 mm et 8 mm, et de préférence encore, entre 1,5 mm et 5 mm.

24. Dispositif selon l'une quelconque des revendications précédentes, **caractérisé en ce que** ledit produit contient une substance aromatique ayant une activité notamment anti-stress ou anti-allergique, ou de confort, favorisant notamment la respiration ou le sommeil.

25. Dispositif selon l'une quelconque des revendications 1 à 24 **caractérisé en ce que** ledit produit est choisi parmi les huiles essentielles, notamment à base de camphre, d'eucalyptus, de lavande, ou de muguet.

## Patentansprüche

1. Inhaliervorrichtung (1), die aus mindestens einem Träger (2) gebildet wird, der ein mindestens einen Wirkstoff aufweisendes Produkt enthält, wobei der Träger eine flüchtige Phase des Wirkstoffs (oder der Wirkstoffe) in Form von Ausdünstungen wiedergeben kann, wobei Mittel (3-6) die Befestigung der Vorrichtung an mindestens einem Punkt ermöglichen, der sich in der Nähe der Nasenlöcher befindet, um eine Inhalation der Ausdünstungen durch die Nase zu ermöglichen, wobei der Träger und die Befestigungsmittel so ausgebildet sind, dass die Ausdünstungen nicht direkt vor oder in Kontakt mit den Nasenlöchern ausgerichtet sind, **dadurch gekennzeichnet, dass** sie Mittel (31, 28, 23, 24) aufweist, um die Dosis der vom Träger (2) wiedergegebenen Inhalationen variieren zu lassen.

2. Vorrichtung nach Anspruch 1, **dadurch gekennzeichnet, dass** die Befestigungsmittel von mindestens einem Abschnitt eines Körpers (20) aus einem gegenüber den Ausdünstungen dichten Material gebildet werden, der den (die) Träger (2) aufnehmen kann.

3. Vorrichtung nach einem der Ansprüche 1 und 2, **dadurch gekennzeichnet, dass** sie zwei Armabschnitte (3, 4) aufweist, die eine Klammer bilden, deren erstes Ende fest mit der Vorrichtung verbunden und deren zweites Ende (5, 6) frei ist, wobei die zweiten Enden (5, 6) einander gegenüberliegen und einen Raum begrenzen, der die Befestigung der Vorrichtung (1) auf dem Nasenrücken, auf Nasenknorpel oder an der einen oder der anderen der die Nasenlöcher begrenzenden Seitenwände ermöglicht, wobei die Armabschnitte (3, 4) sich beim Befestigen der Vorrichtung elastisch auseinander spreizen können.

4. Vorrichtung nach Anspruch 3, **dadurch gekennzeichnet, dass** sie im wesentlichen entgegengesetzt zu einem Mittelpunkt des von den zweiten Enden (5, 6) begrenzten Raums einen Ausschnitt (15) aufweist, der den Armabschnitten (3, 4) eine größere Elastizität verleihen kann.

5. Vorrichtung nach Anspruch 2, **dadurch gekennzeichnet, dass** der Körper (20) eine Aussparung (30) bildet, die den (oder die) Träger (2) aufnehmen kann, wobei der Körper (20) aus einem gegenüber den Ausdünstungen dichten Material besteht und mindestens eine Öffnung (31, 32, 33) aufweist, um die Ausdünstungen durchzulassen.

6. Vorrichtung nach Anspruch 5, **dadurch gekennzeichnet, dass** der Träger aus einer Struktur mit zwei übereinander angeordneten Lagen (34, 35) besteht, oder dass die Vorrichtung zwei übereinander angeordnete Träger aufweist, wobei der erste Träger oder die erste Lage (34) einen ersten Wirkstoff aufweist, der durch eine erste Öffnung (32) diffundiert, die in einer ersten Fläche (25) des Körpers ausgebildet ist, während der zweite Träger oder die zweite Lage (35) einen zweiten Wirkstoff aufweist, der durch eine zweite Öffnung (33) diffundiert, die sich in einer zweiten Fläche (26) des Körpers (20) entgegengesetzt zur ersten, befindet.

7. Vorrichtung nach Anspruch 5 oder 6, **dadurch gekennzeichnet, dass** der (oder die) Träger (2) lösbar in der Aussparung (30) angeordnet ist (sind), um eine nachfüllbare Struktur zu bilden.

8. Vorrichtung nach Anspruch 7, **dadurch gekennzeichnet, dass** der Körper (20) aus einem waschbaren Material besteht.

9. Vorrichtung nach einem der Ansprüche 5 bis 8, **dadurch gekennzeichnet, dass** der Körper (20) aus einem Material hergestellt wird, das aus Polypropylen, Polyethylen, Polyvinylchlorid, den natürlichen, künstlichen oder thermoplastischen Elastomeren ausgewählt wird.

10. Vorrichtung nach einem der Ansprüche 5 bis 9, **dadurch gekennzeichnet, dass** sie eine im wesentlichen flache Struktur mit zwei Hauptflächen (25, 26) und einer Randfläche (27) bildet, wobei die Öffnung in der einen und/oder der anderen der Hauptflächen (25, 26) und/oder in der Randfläche (27) ausgebildet ist.

11. Vorrichtung nach einem der Ansprüche 5 bis 10, **dadurch gekennzeichnet, dass** die Stellung des Trägers (oder der Träger) (2) in der Aussparung so einstellbar ist, dass die Austrittsdosis der Ausdünstungen verändert wird.

12. Vorrichtung nach den Ansprüchen 10 und 11, **dadurch gekennzeichnet, dass** die Randfläche eine Öffnung (31) aufweist, um eine unterschiedlich tiefe Einführung des Trägers (oder der Träger) (2) in die Aussparung (30) zu ermöglichen.

13. Vorrichtung nach einem der Ansprüche 5 bis 10, **dadurch gekennzeichnet, dass** der (oder die) Träger (2) eine ortsfeste Stellung in der Aussparung (30) hat (haben), wobei die Größe mindestens einer Öffnung (28) variabel ist, um die Austrittsdosis der Ausdünstungen zu verändern.

14. Vorrichtung nach den Ansprüchen 10 und 13, **dadurch gekennzeichnet, dass** der Körper einen Boden (23) und einen drehbar am Boden befestigten Deckel (24) enthält, wobei die Winkelstellung des Bodens (23) in bezug auf den Deckel (24) die Größe einer Öffnung (28) bestimmt, die entweder auf der Randfläche (27) oder auf mindestens einer der Hauptflächen (25, 26) der Vorrichtung ausgebildet ist.

15. Vorrichtung nach einem der Ansprüche 1 bis 14, **dadurch gekennzeichnet, dass** der Träger (2) aus einem Element (16) besteht, das eine Struktur bildet, die ein geschlossenes Volumen definiert, welches das Produkt enthalten kann, wobei das Element durch Blasformen aus einem für die Ausdünstungen durchlässigen Material erhalten wird.

16. Vorrichtung nach Anspruch 15, **dadurch gekennzeichnet, dass** das Material aus den Polyethylenen niedriger Dichte, den weichen Polyvinylchloriden oder den Ethylen-Vinylacetatalkohol-Copolymeren (EVA) ausgewählt wird.

17. Vorrichtung nach einem der Ansprüche 1 bis 14, **dadurch gekennzeichnet, dass** der (oder die) Träger (2) aus einem porösen Material besteht (bestehen), das das Produkt in die Tiefe aufnehmen kann.

18. Vorrichtung nach Anspruch 17, **dadurch gekennzeichnet, dass** das Material durch Vakuumsättigung, durch Auffüllen, durch Druck oder durch Kapillarwirkung mit Produkt gesättigt wird.

19. Vorrichtung nach Anspruch 17 oder 18, **dadurch gekennzeichnet, dass** das Material aus den NBR-Schaumstoffen (natural butadiene rubber), den SBR-Schaumstoffen (synthetic butadiene rubber), den Elastomerschaumstoffen, insbesondere aus Polyether-Blockamid, aus Polyurethan, oder aus PEBAX®, aus Filz, Wolle, Cellulose, Holz, Terrakotta, Gips, Keramikmaterialien usw. ausgewählt wird.

20. Vorrichtung nach einem der Ansprüche 1 bis 16, **dadurch gekennzeichnet, dass** der (oder die) Träger aus einer Matrix besteht (bestehen), die aus einem Material besteht, das aus Silicon, Latex, Polyurethan, Gummi ausgewählt wird.

21. Vorrichtung nach einem der vorhergehenden Ansprüche, **dadurch gekennzeichnet, dass** zumindest eine der Flächen (7, 8) des Trägers (2) mit einer Folie (10-13) aus einem Material aus Polyethylen oder Filz bedeckt ist.

22. Vorrichtung nach dem vorhergehenden Anspruch, **dadurch gekennzeichnet, dass** mindestens eine der Folien (12, 13) für die Ausdünstungen undurchlässig und abschälbar ist, um den Durchlass der Ausdünstungen durch die entsprechende Fläche (7, 8) des Trägers (2) zu ermöglichen.

23. Vorrichtung nach einem der vorhergehenden Ansprüche, **dadurch gekennzeichnet, dass** sie eine im wesentlichen flache Struktur bildet und eine Stärke zwischen 0,5 mm und 10 mm, und vorzugsweise zwischen 1 mm und 8 mm, und noch vorzugsweise zwischen 1,5 mm und 5 mm hat.

24. Vorrichtung nach einem der vorhergehenden Ansprüche, **dadurch gekennzeichnet, dass** das Produkt eine aromatische Substanz mit einer angenehmen oder therapeutischen Wirkung, insbesondere stressmindernd oder antiallergisch, die insbesondere den Schlaf oder die Atmung begünstigt, enthält.

25. Vorrichtung nach einem der Ansprüche 1 bis 24, **dadurch gekennzeichnet, dass** das Produkt aus den ätherischen Ölen, insbesondere auf der Basis von Kampfer, Eukalyptus, Lavendel, Thymian oder Maiglöckchen, ausgewählt wird.

## Claims

1. Inhalation device (1) made up of at least one support (2) containing a product which includes at least one active substance, the said support being able to release, in the form of emanations, a volatile phase of the said active substance (or active substances), means (3 - 6) permitting attachment of the device at at least one point situated near the nostrils, in such a way as to permit an inhalation of the said emanations by the nasal route, the support and the attachment means being designed in such a way that the emanations are not oriented directly opposite or in contact with the nostrils, **characterized in that** it comprises means (31, 28, 23, 24) for varying the flow rate of the inhalations released by the support (2).

2. Device according to Claim 1, **characterized in that** the said attachment means are made up of at least one portion of a body (20) made of a material which is impervious to the emanations, and able to receive the support (or supports) (2).

3. Device according to either of Claims 1 and 2, **characterized in that** it includes two arm portions (3, 4) forming a clip, a first end of which is integral with the device, a second end (5, 6) being free, the said second ends (5, 6) being situated opposite one another and delimiting a space able to permit attachment of the device (1) to the bridge of the nose, to the nasal cartilage, or on one or other of the side walls delimiting the nostrils, the said arm portions (3, 4) being able to spread apart elastically during attachment of the device.

4. Device according to Claim 3, **characterized in that**, essentially opposite a median point of the space delimited by the said second ends (5, 6), the device has a notch (15) which is able to confer greater elasticity on the said arm portions (3, 4).

5. Device according to Claim 2, **characterized in that** the said body (20) forms a recess (30) able to receive the support (or supports) (2), the said body (20) being made of a material impervious to the said emanations, and including at least one opening (31, 32, 33) to allow the said emanations to pass through.

6. Device according to Claim 5, **characterized in that** the support consists of a structure of two superposed layers (34, 35), or **in that** the device comprises two superposed supports, the first support or the first layer (34) comprising a first active substance diffusing through a first opening (32) formed in a first face (25) of the body, the second support or the second layer (35) comprising a second active substance diffusing through a second opening (33) formed in a second face (26) of the body (20), opposite the first one.

7. Device according to Claim 5 or 6, **characterized in that** the support (or supports) (2) is (are) mounted in a removable manner in the recess (30) in such a way as to form a reloadable structure.

8. Device according to Claim 7, **characterized in that** the said body (20) is made of a washable material.

9. Device according to any one of Claims 5 to 8, **characterized in that** the body (20) is made of a material chosen from among polypropylenes, polyethylenes, polyvinyl chlorides, and natural, synthetic or thermoplastic elastomers.

10. Device according to any one of Claims 5 to 9, **characterized in that** it forms a substantially flat structure which includes two main faces (25, 26) and an edge (27), the said opening being formed in one and/or the other of the said main faces (25, 26) and/or in the edge (27).

11. Device according to any one of Claims 5 to 10, **characterized in that** the position of the support (or supports) (2) in the recess can be adjusted in such a way as to modify the output rate of the said emanations.

12. Device according to Claims 10 and 11, **characterized in that** the edge includes an opening (31) allowing the support (or supports) (2) to be inserted to a variable depth into the recess (30).

13. Device according to any one of Claims 5 to 10, **characterized in that** the support (or supports) (2) is (are) in a fixed position in the recess (30), the size of at least one opening (28) being variable in such a way as to modify the output rate of the said emanations.

14. Device according to Claims 10 and 13, **characterized in that** the body includes a base (23) and a lid (24) mounted in rotation on the base, the angular position of the base (23) in relation to the lid (24) determining the size of an opening (28) formed either on the edge (27) or on at least one of the main faces (25, 26) of the device.

15. Device according to any one of Claims 1 to 14, **characterized in that** the support (2) consists of an element (16) forming a structure which defines a closed volume which can contain the product, the said element being obtained by blow-moulding a material which is permeable to the said emanations.

16. Device according to Claim 15, **characterized in that** the said material is chosen from among low-density polyethylenes, flexible polyvinyl chlorides, or copolymers of ethylene and vinyl acetate (EVA).

17. Device according to any one of Claims 1 to 14, **characterized in that** the support (or supports) (2) is (are) made of a porous material which is able to deeply absorb the product.

18. Device according to Claim 17, **characterized in that** the said material is saturated with product, by saturation under vacuum, by swelling, by pressure, or by capillarity.

19. Device according to Claim 17 or 18, **characterized in that** the said material is chosen from among NBR (natural butadiene rubber) or SBR (synthetic butadiene rubber) foams, elastomer foams, in particular of polyether block amide, polyurethane, or PEBAX®, felt, wool, cellulose, wood, terracotta, plaster or ceramics.

20. Device according to any one of Claims 1 to 16, **characterized in that** the support (or supports) is (are) made up of a matrix consisting of a material chosen from among silicones, latices, polyurethanes or gums.

21. Device according to any one of the preceding claims, **characterized in that** at least one of the faces (7, 8) of the support (2) is covered with a sheet (10 - 13) of a polyethylene or felt material.

22. Device according to the preceding claim, **characterized in that** at least one of the sheets (12, 13) is impervious to the said emanations and can be peeled off so as to permit the passage of the emanations through the corresponding face (7, 8) of the support (2).

23. Device according to any one of the preceding claims, **characterized in that** it forms a substantially flat structure and has a thickness of between 0.5 mm and 10 mm, preferably of between 1 mm and 8 mm, and still more preferably of between 1.5 mm and 5 mm.

24. Device according to any one of the preceding claims, **characterized in that** the said product contains an aromatic substance having an activity in particular against stress or against allergies, or providing comfort, assisting in particular in breathing or sleep.

25. Device according to any one of Claims 1 to 24, **characterized in that** the said product is chosen from among essential oils, particularly based on camphor, eucalyptus, lavender or lily of the valley.
